# EUROPÄISCHE PATENTANMELDUNG

(11) **EP 1 110 520 A1**
(43) Veröffentlichungstag der Anmeldung: **27.06.2001**
(21) Anmeldenummer: 00124866.5
(22) Anmeldetag: 15.11.2000
(51) Int. Cl.: A61F 9/007, A61B 17/32

(54) **Chirurgisches Schneidinstrument**

(30) Priorität: 21.12.1999 CH 233599
(71) Anmelder: H.P. Braem AG, 9402 Mörschwil (CH)
(72) Erfinder: Bräm, Hans Peter, 9402 Mörschwil (CH)
(74) Vertreter: Römpler, Aldo

(57) **Zusammenfassung**

Das Chirurgisches Schneidinstrument weist zwei voneinander lösbare Teile auf, nämlich ein Handgriff (A) und ein Schneideteil (B). Die Verbindung zwischen dem Handgriff (A) und dem Schneideteil (B) ist derart ausgebildet, dass der elektrische bzw. elektromagnetische Antrieb (7 - 10) im Handgriff (A) angeordnet ist. Die Verbindung zur Schneide (17) erfolgt über eine Kupplung (1). Das Schneideteil (B) kann nach jeder Operation weggeworfen, der Handgriff (A) wiederverwendet werden. Dadurch wird ein Höchstmass an Sauberkeit und eine Verringerung des Anteils an wegzuwerfendem Material erreicht. Der linear bewegliche Magnetkern (10) des Antriebs ist vorzugsweise so ausgebildet, dass er kürzer als die Wicklung (7) ist. Der Schnitt erfolgt dadurch rascher und präziser, wodurch die Genauigkeit des chirurgischen Eingriffs erhöht wird.

## Beschreibung

Die vorliegende Erfindung betrifft ein chirurgisches Schneidinstrument mit mindestens einer eine Hubbewegung ausführenden Schneide, wobei der Antrieb elektrisch oder elektromagnetisch erfolgt.

In der Ophthalmochirurgie, insbesondere für chirurgische Eingriffe am Glaskörper des Auges, werden Schneidinstrumente verwendet, die eine in einer Kanüle längsverschieblich geführte Schneide aufweisen, die durch einen Antrieb in Hubbewegungen versetzt wird. Die bekannten Kanülen weisen ein Kanülenrohr mit kreisrundem Querschnitt auf. Am in das Auge einzuführenden Ende ist am Umfang der Kanüle eine Öffnung angebracht, die als Schneid- und Ansaugöffnung oder auch als Spülöffnung dient. Das andere Ende ist mit einem Handgriff verbunden, das seinerseits über eine flexible Leitung, bzw. flexible Leitungen, lösbar an ein entsprechendes Gerät angeschlossen ist. Der Schnitt erfolgt dadurch, dass das zu schneidende Material zwischen der Schneide und der Wand der Kanülenöffnung abgetrennt wird. Der im Inneren der Kanüle verbleibende, abgeschnittene Teil, wird durch Absaugen abgeführt. Dieser Vorgang wiederholt sich kontinuierlich bis der operative Eingriff abgeschlossen ist. Die in der Kanüle angeordnete Schneide wird entweder hydraulisch, elektrisch oder elektromagnetisch bewegt. In der elektromagnetischen Ausführung erfolgt die Hubbewegung zumeist entgegen der Kraft einer Feder. Dieses Schneidinstrument ist entweder nur einmal zu verwenden oder zur Wiederverwendung zu sterilisieren. Bekannt ist es auch, die Kanüle vom Handgriff lösbar als Wegwerfartikel auszubilden, wobei sich jedoch Probleme beim zuverlässigen Sterilisieren der übrigen Teile ergeben. Insbesondere bei den elektromagnetisch angetriebenen Schneidinstrumenten ist daher das ganze Instrument, einschliesslich des die elektromagnetische Spule enthaltenden Handgriffes, als Wegwerfartikel vorgesehen. Es versteht sich von selbst, dass diese Lösung zwar für jede Operation ein einwandfrei keimfreies Schneidinstrument gewährleistet, jedoch teuer ist. Zudem können die bekannten, elektromagnetisch angetriebenen Schneidinstrumente nur mit maximal 10 Hz arbeiten.

Der Erfindung liegt die Aufgabe zugrunde, ein chirurgisches Schneidinstrument mit mindestens einer elektrisch oder elektromagnetisch in eine Hubbewegung versetzbaren Schneide so zu verbessern, dass der technische Materialaufwand für einen operativen Eingriff verringert und daher die Kosten gesenkt werden können. Ferner soll auch die Schneidwirkung verbessert werden.

Das erfindungsgemässe Schneidinstrument entspricht den kennzeichnenden Merkmalen des Patentanspruchs 1. Weitere vorteilhafte Ausbildungen des Erfindungsgegenstandes gehen aus den abhängigen Patentansprüchen hervor.

Nachfolgend wird ein bevorzugtes Ausführungsbeispiel des erfindungsgemässen Schneidinstrumentes anhand der Zeichnung näher beschrieben.
- Fig. 1: zeigt einen Schnitt durch den Handgriff des Schneidinstrumentes,
- Fig. 2: zeigt einen entsprechenden Schnitt durch das Schneideteil.

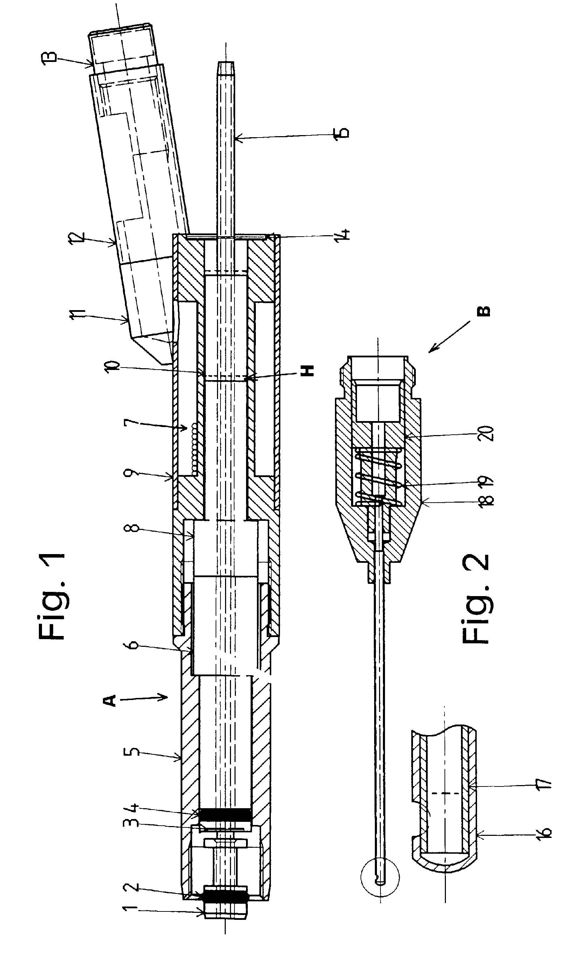

Das chirurgische Schneidinstrument weist einen Handgriff A nach Fig. 1 auf, der als zu sterilisierendes, mehrfach verwendbares Teil vorgesehen ist. In dieses ist ein Schneideteil B nach Fig. 2 einsteckbar, das als Einweg-bzw. Wegwerfartikel gedacht ist. Der Antrieb ist elektromagnetisch.

Der Handgriff A weist eine Kupplung 1 auf, die als Dichtung mit einem O-Ring 2 versehen ist. Hier ist das Schneideteil B nach Fig. 2 - wie später noch erläutert - aufsteckbar. In der dem operativen Ende entgegengesetzten Richtung schliessen sich im vorliegenden Beispiel eine Membrane 3 und ein weiterer O-Ring 4 an. Das Griffrohr des Handgriffs A ist mit 5 bezeichnet, ein mit einem Anker 8 verbundenes Distanz-Teil 6. Der elektromagnetische Antrieb weist - wie bekannt - eine auf eine Spule aufgewickelte Wicklung 7, den Anker 8, einen Magnetmantel 9 und einen Magnetkern 10 auf. Auf die Besonderheiten des Magnetkerns 10 wird später eingegangen. Das Distanz-Teil 6 reicht mit einer im Querschnitt verjüngten Verlängerung bis in den Bereich der Membrane 3. Der Anker 8, ebenfalls mit einer im Querschnitt verjüngten Verlängerung, bis zum längsverschieblichen Magnetkern 10. Die Teile 11 - 13 gehören zu einem Anschluss für eine Spülleitung. Es handelt sich um einen Kabelanschluss 11, einem Rohr 12 und einer Mutter 13. Am rückwärtigen Anschlussbereich des Handgriffs A ist eine weitere, der Abdichtung dienende Membrane 14 angeordnet. Durch die Längsmitte des Handgriffs A ist ein Aspirationsrohr 15 geführt, durch welches das abgeschnittene Material sowie sonstige Operations-Rückstände und Flüssigkeit absaugbar sind. Das Aspirationsrohr 15 ist durch die ganze Länge des Handgriffs A geführt und verbindet das Schneideteil B über eine am Handgriff A anzuschliessenden Leitung an ein entsprechendes, bekanntes Gerät.

Das Schneideteil B weist eine Kanüle 16 auf. An derem Umfang ist im Bereich des in das Auge einzuführenden Endes eine Öffnung angebracht, die als Schneid- und Ansaugöffnung oder auch als Spülöffnung dient. Siehe hierzu das vergrössert dargestellte Ende der Kanüle 16. Im Innern der Kanüle 16 ist die rohrartig ausgebildete Schneide 17 längsverschieblich angeordnet. Diese ist in ihrer vorderen Endposition dargestellt; die hintere Position ist gestrichelt angedeutet. Zwischen diesen beiden Endpositionen erfolgt die eingangs erwähnte Hubbewegung. Dadurch, dass die Schneide 17 an der Öffnung vorbeigeführt wird, kann das in die Öffnung hinein ragende Material abgeschnitten und durch den leeren Innenraum der Schneide 17 abgeführt werden.

Die Kanüle 16 ist in das als Kappe ausgebildete Gehäuse 18 des Schneideteils B eingesteckt. Im rohrartigen Hohlraum dieses Gehäuses 18 ist eine Feder 19 angeordnet, durch deren Kraft ein längsverschiebliches, bewegliches Element 20 von der Kanüle 16, bzw. von der Instrumentenspitze weggedrückt wird. Das Element 20 ist in seinem Inneren hohl. Das im Inneren der Kanüle 16 bzw. der rohrartigen Schneide 17 befindliche, abgeschnittene Material wird durch dieses Innere zum Aspirationsrohr 15 geführt bzw. abgesaugt. Das Element 20 weist in diesem Beispiel zur Kanüle 16 hin zwei Verjüngungen seines Umfangs auf. Auf die erste ist die Feder 19 aufgesteckt. Die zweite dient, in einer entsprechenden Querschnittsverringerung des Gehäuses 18, als zweite Führung. Die Kanüle 16 ist ortsfest mit dem Gehäuse verbunden. Die in deren Inneren geführte Schneide 17 ist dagegen mit dem Element 20 verbunden, bzw. in dessen Innenquerschnitt eingesteckt. Dadurch macht die Schneide 17 die Hubbewegungen des Elementes 20 mit. An seinem der Kanüle 16 abgewandten Ende weist das Element 20 eine buchsenartige Aufnahme für die Kupplung 1 des Handgriffs A auf.

Das Gehäuse 18 des Schneideteils B ist mit einem Aussengewinde versehen. Das die Kupplung 1 mit Abstand umgebende Ende des Griffrohres 5 des Handgriffs A weist an seiner Innenwandung ein entsprechendes Innengewinde auf. Das Gehäuse 18 und das Griffrohr 5, und damit das Schneideteil B und der Handgriff A, lassen sich somit durch Verschraubung fest miteinander verbinden. Nur die in deren Inneren liegenden Teile 1, 2, 10, 15 sowie 17, 19, 20 sind beweglich.

Durch den elektromagnetischen Antrieb wird, bei Beaufschlagung mit elektrischem Strom, der das Aspirationsrohr 15 umgebende und mit diesem ortsfest verbundene Magnetkern 10 in Richtung des Schneideteils B gedrückt. Mit dem Magnetkern 10 bewegt sich also auch das Aspirationsrohr 15, welches verschiebbar durch das ortsfeste Distanz-Teil 6 und den Anker 8 hindurch geführt ist. In der Zeichnung ist der Magnetkern 10 in seiner vorderen Endposition dargestellt, der Hub H ist gestrichelt angedeutet. Entsprechend bewegt sich auch das am Ende des Aspirationsrohres 15 angeordnete Anschlussteil mit der Kupplung 1.

Da diese Kupplung 1 in die zuvor genannte Aufnahme des beweglichen Elementes 20 des Schneideteils B eingesteckt ist, wird dieses - und damit auch die Schneide 17 - in Richtung der Instrumentenspitze gedrückt. Die Feder 19 wird dadurch zusammengedrückt. Beim Ausschalten der Magnetkraft drückt die Feder 19 das Element 20 und die Schneide 17 wieder zurück in deren hintere Position. Dieser Hubzyklus erfolgt automatisch, bei den bisher bekannten elektromagnetischen Schneidinstrumenten bei 10 Hz mit annähernd 600 Hubbewegungen pro Minute. Dieser sich rasch wiederholende Schneidevorgang kann vom Chirurgen ein- und ausgeschaltet werden.

Für die Erfindung massgebend ist, dass das Schneidinstrument aus mindestens zwei voneinander lösbaren Teilen besteht, nämlich dem Handgriff A mit dem elektromagnetischen Antrieb 7 - 10 und dem Schneideteil B mit der in der Kanüle 16 geführten Schneide 17. Die lösbare Verbindung dieser beiden Teilen A und B ist so gestaltet, dass sie - was die Kraftübertragung betrifft - zwischen den linear beweglichen Teilen des elektromagnetischen Antriebs 10 und 15, bzw. einer an diese angeschlossenen Kupplung 1, und der Schneide 17 erfolgt, wobei das Schneideteil B ein mit der Schneide 17 verbundenes Element 20 aufweist, zur Übertragung der linearen Antriebskraft auf die Schneide 17.

Hierdurch ist sichergestellt, dass der dem operativen Eingriff dienende und nach jeder Operation wegzuwerfende Schneideteil B einwandfrei steril ist. Dadurch, dass das Schneideteil B ein als Frontkappe ausgebildetes Gehäuse 18 aufweist, ist zwischen dem Handgriff A und dem Operationsbereich ein steriles Schutzschild vorhanden. Der grösste Teil A des chirurgischen Schneidinstrumentes ist, nach entsprechender Sterilisierung, vorzugsweise in einem Autoklaven, wieder verwendbar. Der bei jeder Operation anfallende Materialverlust ist damit sehr gering gehalten.

In weiterer Verbesserung dieses Schneidinstrumentes weist der elektromagnetische Antrieb eine Besonderheit auf. Der Magnetkern 10 ist so ausgebildet, dass er im Verhältnis zur Wicklung 7 kurz ist. In herkömmlichen Antrieben erstreckt sich der Magnetkern 10 über die ganze Länge der Wicklung 7. Hier reicht er nur bis annähernd zur Mitte der Wicklung 7. Dadurch wird die zu bewegende Masse verringert. Bei Einschaltung des elektrischen Stromes setzt sich der Magnetkern 10 früher in Bewegung. Diese Bewegung ist auch schneller. Konkret bedeutet dies, dass der Hubzyklus mit 30 Hz bei annähernd 1'800 Hubbewegungen pro Minute liegen kann, d.h. bis zum Dreifachen des bisher möglichen. Hierdurch wird die Schnittqualität und infolgedessen die Operationsgenauigkeit erheblich verbessert.

Im Rahmen der Erfindung sind selbstverständlich auch andere Ausführungen als das vorgehend beschriebene, bevorzugte Ausführungsbeispiel möglich. So wäre es beispielsweise denkbar, die gleiche Verbindung zwischen dem Handgriff A und dem Schneideteil B auch bei einem Schneidinstrument vorzusehen, bei dessen elektrischen Antrieb die Kraft einer rotierenden Antriebswelle durch eine bekannte Taumelscheibe, siehe z.B. US-Patent Nr. 5'833'643, in eine Hubbewegung umgesetzt wird. Im Schneideteil B wäre in diesem Fall keine Feder 19 erforderlich, da durch diesen Antrieb sowohl die Vorwärts- als auch die Rückwärtsbewegung der Schneide 17 erfolgt.

Die Kupplung 1 sowie die entsprechende Aufnahme am Schneideteil B können auch anders ausgebildet werden. Wichtig ist nur, dass eine einfache, rasch lösbare und dennoch dichte Verbindung zwischen den beweglichen Teilen 10, 15 des Antriebs einerseits und der Schneide 17, bzw. einem mit dieser verbundenen Element 20, sichergestellt ist.

Das vorliegende Schneidinstrument bringt mehrere entscheidende Vorteile mit sich. Es wird sowohl ein Höchstmass an Sauberkeit als auch an Genauigkeit des chirurgischen Eingriffs erreicht. Die Gefahr von postoperativen Komplikationen wird dadurch vermindert. Dies bei einer Verringerung des Anteils an wegzuwerfendem Material und der damit verbundenen Kosten.

## Patentansprüche

1. Chirurgisches Schneidinstrument mit mindestens einer eine Hubbewegung ausführenden Schneide, wobei der Antrieb elektrisch oder elektromagnetisch erfolgt, dadurch gekennzeichnet, dass es aus mindestens zwei voneinander lösbaren Teilen (A und B) besteht, wobei eine lösbare Verbindung zwischen einem Handgriff (A) und einem Schneideteil (B) derart ausgebildet ist, dass der elektrische oder elektromagnetische Antrieb (7 - 10) im Handgriff (A) angeordnet ist und das zum Schneideteil (B) gerichtete, freie Ende des Handgriffs (A) und der darin angeordneten, linear beweglichen Teile (10, 15) des Antriebs sowie das Schneideteil (B) so ausgebildet sind, dass die Antriebskraft an eine Schneide (17) im Schneideteil (B) übertragbar ist.

2. Chirurgisches Schneidinstrument nach Anspruch 1, dadurch gekennzeichnet, dass am zum Schneideteil (B) gerichteten, freien Ende der linear beweglichen Teile (10, 15) des Antriebs eine Kupplung (1) zum Anschluss an das Schneideteil (B) vorgesehen ist und das Schneideteil (B) Mittel (20) aufweist, zur Übertragung der von der Kupplung (1) weitergegebenen Antriebskraft an die Schneide (17).

3. Chirurgisches Schneidinstrument nach Anspruch 2, dadurch gekennzeichnet, dass im Schneideteil (B) als Mittel zur Übertragung der Antriebskraft ein längsverschiebliches Element (20) vorgesehen ist, das einerseits an die Kupplung (1) des Handgriffs (A) anschliessbar ist und andererseits mit der Schneide (17) in Wirkverbindung steht.

4. Chirurgisches Schneidinstrument nach Anspruch 3, dadurch gekennzeichnet, dass im Schneideteil (B) eine Feder (19) vorgesehen ist, durch deren Kraft das längsverschiebliche Element (20) in Richtung des Handgriffs (A) gedrückt wird, so dass die Schneide (17) durch den Antrieb nur entgegen der Kraft dieser Feder (19) in die Schneideposition drückbar ist.

5. Chirurgisches Schneidinstrument nach einem der Ansprüche 3 oder 4, dadurch gekennzeichnet, dass das längsverschiebliche Element (20) in einem Gehäuse (18) angeordnet ist, das vorzugsweise als Kappe ausgebildet ist, an welchem eine Kanüle (16) befestigt ist, in deren Inneren die Schneide (17) geführt ist.

6. Chirurgisches Schneidinstrument nach Anspruch 5, dadurch gekennzeichnet, dass das längsverschiebliche Element (20) an seinem dem Handgriff (A) zuzuwendenden Ende Befestigungsmittel, z.B. eine buchsenartige Aufnahme, für die Kupplung (1) der beweglichen Teile (10, 15) des Antriebs des Handgriffs (A) aufweist.

7. Chirurgisches Schneidinstrument nach einem der Ansprüche 1 - 6, dadurch gekennzeichnet, dass der Handgriff (A), vorzugsweise an einem Griffrohr (5), und das Schneideteil (B), vorzugsweise an einem Gehäuse (18), je ein Gewinde aufweisen, zu deren lösbaren Verbindung.

8. Chirurgisches Schneidinstrument nach einem der Ansprüche 1 - 7, gekennzeichnet durch einen elektromagnetischen Antrieb dessen linear beweglicher Magnetkern (10) so ausgebildet ist, dass er kürzer als die um eine Spule gewickelte Wicklung (7) ist, mit dem Zweck, die zu bewegende Masse zu verringern und eine höhere Hub- bzw. Schneidfrequenz zu erreichen.

9. Chirurgisches Schneidinstrument nach Anspruch 8, dadurch gekennzeichnet, dass der Magnetkern (10), in Arbeitsrichtung von hinten gesehen, bis annähernd zur Hälfte der Wicklung (7) reicht.

10. Chirurgisches Schneidinstrument nach Anspruch 8 oder 9, dadurch gekennzeichnet, dass der Antrieb so ausgelegt ist, dass der Hubzyklus mit annähernd 30 Hz bei annähernd 1'800 Hubbewegungen pro Minute liegt.

11. Chirurgisches Schneidinstrument nach einem der Ansprüche 8 - 10, dadurch gekennzeichnet, dass der Magnetkern (10) fest mit einem vorzugsweise als Aspirationsrohr (15) ausgebildeten Teil verbunden ist, dessen zum Schneideteil (B) gerichtetes, freies Ende Mittel, z.B. eine Kupplung (1), aufweist, zur Übertragung der Antriebskraft an die Schneide (17) im Schneideteil (B).
